# EUROPEAN PATENT SPECIFICATION

(11) **EP 4 201 380 B1**
(45) Date of publication and mention of the grant of the patent: **17.04.2024**
(21) Application number: 22210475.4
(22) Date of filing: 30.11.2022
(51) Int. Cl.: A61F 5/01

(54) **ANKLE-FOOT ORTHOPAEDIC ORTHOSIS**
ORTHOPÄDISCHE FUSSGELENKORTHESE
ORTHÈSE ORTHOPÉDIQUE CHEVILLE-PIED

(30) Priority: 22.12.2021 LT 2021028
(43) Date of publication of application: 28.06.2023
(73) Proprietor: UAB "Ortho Baltic", 51124 Kaunas (LT)
(72) Inventor: Kusvidas, Valentinas, 49306 Kaunas (LT); Areskevicius, Linas, 55333 Uzusaliai village, Jonava distr. (LT); Abakevicius, Gytis, 46383 Kaunas (LT)
(74) Representative: Pakeniene, Ausra

(56) References cited:
- DE-A1-102012 011 466
- DE-A1-102013 019 079
- US-A1- 2011 105 969
- US-A1- 2021 244 603

## Description

### Technical field

The invention belongs to the field of medicine - conservative treatment of the ankle joint of the leg, namely, splinting or immobilisation, the purpose of which is to control and stabilise the ankle joint, seeking to improve the pathological gait.

### Background art

European patent No. EP2858605B1 discloses an ankle-foot orthosis, made of a lightweight and strong carbon composite. The orthosis has a sole and a calf section which adheres to the calf on the ventral side and a spring section on the medial part of the orthosis, which is connected to the footplate. This orthosis has both functional disadvantages and issues when it comes to adapting it to the needs of a specific patient (i.e., problems related to the personalisation of the orthosis). The design of the orthosis does not allow for changes in the height and angular position of the support against the calf. It is difficult to fit corrective shoe inserts to such an orthosis as the structural part, which acts as a spring section, is located in the medial part of the orthosis. This localization of the spring section causes the medial part of the footplate and the ankle bone to rub against the structural elements of the orthosis during the transition from one step phase to the next, especially in the case of hyperpronation. This often causes pain, swelling of the soft tissues adjacent to the orthosis and the formation of ulcers.

The orthosis spring widens as it transitions into both the foot and the calf support parts. This makes it fully stabilised and stiff in the upper part of the orthosis. The spring flares in the sagittal plane into the calf support in the frontal plane, making a curve towards the medial side of the foot. This sagittal extension of the spring limits its flexion to a few centimetres below the tibial support. In addition, the spring forms a cylindrical convexity in the calf support, which is fully stiff, as it transitions into the calf support. Therefore, the orthosis spring does not have flexibility in the sagittal plane, which concentrates the load on the front part of the lower leg in a narrow localization and does not ensure efficient energy storage and release from heel rise to toe off in the stance phase.

The overall height of this orthosis and, at the same time, the height of the calf support are not adjustable, which prevents the selection of the optimal proportion of the calf height in the cases of instability of the knee and increased joint looseness. The applicability of the orthosis (adaptability to specific patients according to their anatomical structure and clinical indications) is therefore considerably reduced. In addition, the structure of the calf support does not allow for adjustment of the angle of the support against the calf, and is non-breathable, which further complicates the adaptation of the orthosis and causes perspiration, thereby increasing the risk of rubbing and ulcer formation.

European patent No. EP1114626B1 discloses an ankle-foot orthosis made of lightweight and strong carbon composite. The orthosis has a sole and a calf section which is ventrally adjacent to the calf and laterally connected to the footplate by a strut. The disadvantage of this orthosis is the structure of the strut itself, which is immobile with respect to the sole in the anterior-posterior axis. This design does not create a spring mechanism effect as the footplate transitions from the heel strike phase to the terminal stance phase. The geometry of the strut is therefore not adapted to the accumulation and release of energy during the stride. In addition, the structure of the orthosis is not stable in the coronal plane, so that during the stride, as the footplate transitions from the heel strike phase to the terminal stance phase and the orthosis moves forward, the forces acting on the orthosis, which are concentrated at the bottom of the strut, at the junction with the sole, cause the orthosis to deform. The sole of the orthosis includes a tough, rigid arch support which prevents an even redistribution of these forces over the whole area of the sole, thus rendering the whole structure of the orthosis rigid. This significantly reduces the wearing comfort and durability of the orthosis, increases the risk of fracture of the sole and the strut, and minimises the accumulation and release of energy during stepping. On the lateral side, steeply downward, sole section-connecting support makes it difficult to fit corrective inserts moulded on the sole and increases the risk of lateral tarsus bone injury. The calf support of the orthosis is made of a rigid carbon fibre composite which does not adapt to the shape of the person's calf and therefore, in order to increase the adaptability of the orthosis, the support area is made much narrower (smaller) and does not provide a secure fastening of the calf. The homogeneous design makes the calf support completely non-breathable, which causes perspiration and may increase the risk of rubbing and ulcers. The structure of the calf support, which is made of a carbon fibre composite, is structurally an integral part of the strut. This does not allow the height and angle of the support against the calf to be adjusted, which severely limits the adaptability to the patient's anatomical features and clinical indications.

There are also a generic ankle-foot orthosis described in the patent No. DE 10 2013 019079 A1 which discloses the preamble of claim 1, as well as similar product available in the market *"FH250 Matrix Max"* from *"Trulife"* - these are also made of a lightweight and tough carbon composite, with a sole and a calf section which is ventrally adjacent to the calf and laterally connected to the footplate by a strut, which originates slightly anterior of the ankle. The main advantage of the orthosis is the adjustability of the height of the calf support, but the structure of the orthosis does not allow for any adjustment of the angle of support and the overall design of the orthosis has high flexural rigidity in the direction of flexion and extension of the ankle joint the calf support is closed and non-breathable, and the overall design of the orthosis is rigid and does not allow for the maximal storage and release of the energy generated during the stride. The calf support is closed and non-breathable, having just three holes for each of the buckles which would not provide sufficient ventilation as opposed to 3D printed open-worked structure.

US9901475B2 discloses an ankle-foot orthopaedic orthosis. The main parts of the device, i.e., the frame and the calf support, are in a single section, the whole orthosis is made of only one type of material (composite), and the fastening solution is a standard solution used in technical orthopaedic applications. The main disadvantages of the prototype are the lack of functionality and personalisation options.

### Brief description of the invention

The ankle-foot orthopaedic orthosis according to the invention comprises a frame made of a lightweight and tough carbon composite and the added calf support section made of polyamide, such as, but not limited to, *Nylon 12,* using additive manufacturing techniques, such as, but not limited to, SLS. The frame, in turn, consists of two functional parts: a sole (or footplate) and a spring part. The lower part of the spring is curved and connected to the sole on the dorsal side of the foot. As the spring rises upwards, it transitions smoothly in the sagittal plane into a curved spring section that stores and returns energy in the anterior-posterior direction, and in the flat upper part, which is connected to the calf support via mechanisms adjusting the height and angle of the calf support. The calf support is open-worked and made of a biocompatible material (polyamide), using three-dimensional printing technology. It is attached to the leg by calf straps secured by buckles made using three-dimensional printing technology. The fastening mechanism of the buckles is incorporated into the overall structure of the calf support.

The footplate of the orthosis is manufactured using a carbon fibre continuous layering technology. This ensures the flexibility of the sole and the even distribution of forces over the entire footplate area.

The sole connects on the lateral side to the energy-accumulating curved spring section of the frame, forming the middle part of the frame. On the lateral side of the foot, the frame (mid-frame or curved spring section) has a crescent-shaped bow with a convexity on the inside and a concavity on the outside, to maintain the plane of the spring. The bending of the spring runs along its entire length of curved spring section through the concave edge of the spring frame. This design limits the medial-lateral bending of the orthosis and its rotation with respect to the calf support and the foot during the stride phase. Thanks to the even stiffness of the spring, this crescent-shaped bend bypasses the tarsus bone not only at rest but also during the stride phase. At the same time minimises the likelihood of the orthosis coming into contact with the tarsus joint and stores the energy of the stride as the footplate transitions from the heel strike phase to the terminal stance phase.

The curve of the orthosis frame, acting as a spring mechanism, distributes the transfer of energy over time to the sole of the orthosis and its return at the end of the stride more evenly, thus ensuring longer durability of the orthosis compared to the prototype. This spring mechanism eliminates instability of the orthosis in the coronal plane, significantly reducing the likelihood of ankle joint trauma when wearing the orthosis. In turn, the connection of the spring section to the sole of the frame facilitates the fitting of customised footplate inserts. The upward curve of the frame is directed ventrally and transitions smoothly into the coronal plane flat structural element (i.e., the flat spring section). This frame element serves for fixing the calf support and adds to the cushioning properties of the dorsal curve, so that the overall design of the entire carbon composite frame, in contrast to the prototype, ensures that the energy is stored and transferred evenly throughout the support area and is returned to the user at the end of the stride. The shape of the frame ensures an anatomically correct tilt of the orthosis during the stride and prevents it from rotating in the direction of the central axis of the calf.

At the upper ventral part, the calf support is attached to the orthosis frame via height and support angle adjusters. The support is 3D printed and has an openwork structure to minimise the likelihood of perspiration at the contact surface between the orthosis and the calf. It is attached to the flat upper spring part with screws. Height and support angle adjusters, independent of the position of the calf support and the sole, allow the support angle to be adjusted in the dorsal-ventral direction and the support position to be adjusted in the superior-inferior direction in respect to the foot. This functionality is unique and is not offered by any other analogue on the market. It allows for a broader personalisation of the orthopaedic ankle-foot orthosis to the individual patient, taking into account anatomical features and clinical indications, specifically but not limited to limited knee joint angles, calf deformities, limb shortening, and different person's physiological proportions.

The upper part of the orthosis, the calf support, as well as the fastening and adjustment mechanisms, are made by employing the additive manufacturing techniques, using SLS technology from, but not limited to, polyamide such as nylon PA12. This material is lightweight, flexible, abrasion-resistant, biocompatible and extremely durable. These characteristics, even with a large support area, allow the support to automatically adapt to the shape of the calf over a very wide range. Therefore, the same orthosis can be adapted to both thick and thin calves.

The shape of the calf support is based on the principle of the shape of butterfly wings. This shape ensures better fastening of the orthosis in both the lateral and medial part of the leg than the counterparts available on the market and their closest prototype. The orthosis is attached to the calf by textile straps which are passed through openings in the calf support and through specially designed buckles. The buckle fasteners are incorporated directly into the design of the calf support and form part of the orthosis as a whole. The structure of the buckles ensures that the orthosis is securely locked in place, even in the event of breakage of the locking mechanism or accidental release of the buckle, for example, in the event of striking surrounding objects.

This locking solution increases the support area of the orthosis against the calf and prevents movement of the calf independently of the orthosis. As the calf support, together with the carbon fibre (composite) frame, which includes the sole of the orthosis, forms a single structure, the person's leg is not able to move in any direction other than the direction of flexion of the spring section of the frame during the stride. This ensures that the ankle joint moves in the biomechanically correct direction.

### Brief description of the drawings

Features of the invention believed to be novel and inventive are set forth with particularity in the appended claims. The invention itself, however, may be best understood by reference to the following detailed description of the invention, which describes exemplary embodiments, given in non-restrictive examples, of the invention, taken in conjunction with the accompanying drawings, in which:

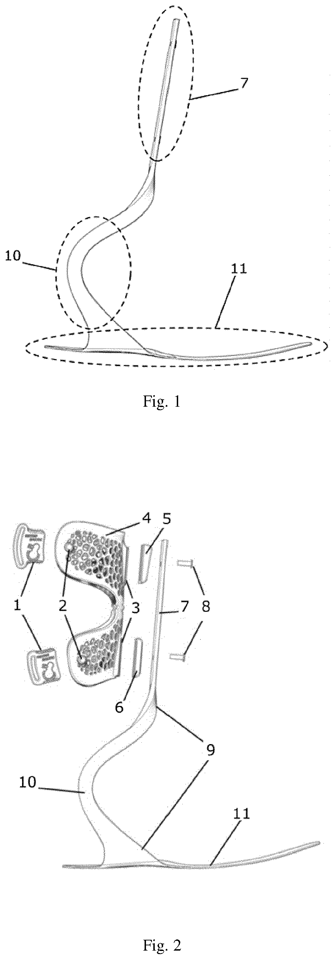

Fig. 1 shows structural elements of the orthosis frame;
Fig. 2 shows lateral view of the orthosis;

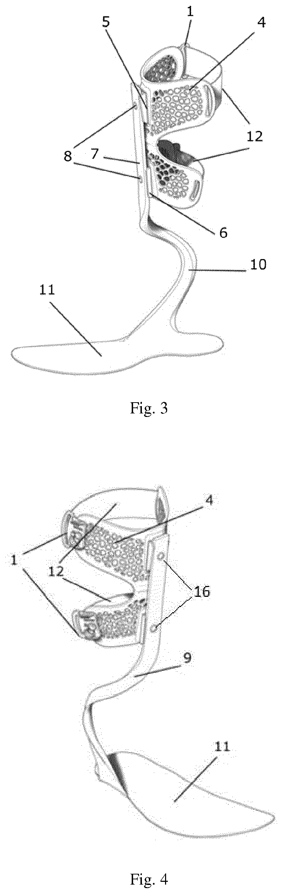

Fig. 3 shows medial view of the orthosis;
Fig. 4 shows perspective view of the orthosis;

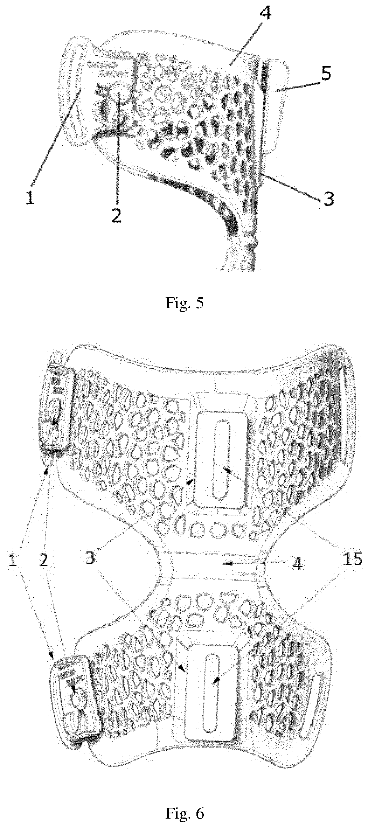

Fig. 5 shows the main elements of the calf support set;
Fig. 6 shows calf support;

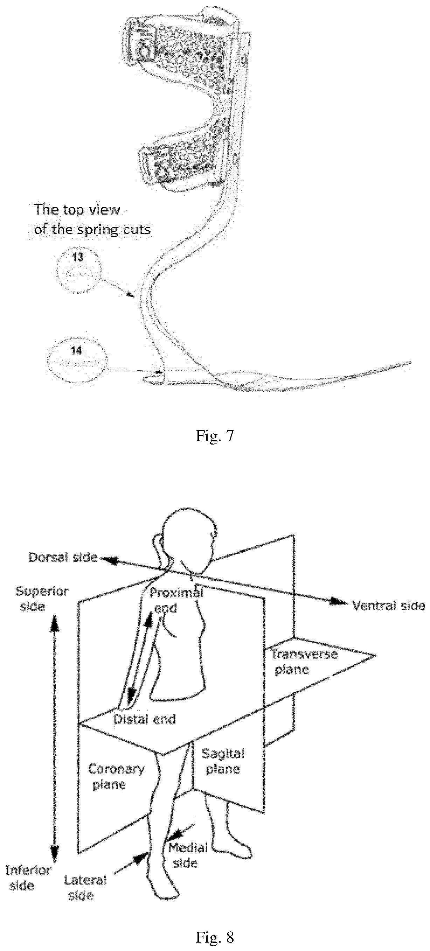

Fig. 7 shows cuts of the spring section;
Fig. 8 shows explanatory diagram of planes and directions.

Preferred embodiments of the invention will be described herein below with reference to the drawings. Each figure contains the same numbering for the same or equivalent element.

### Detailed description of the invention

The ankle-foot orthopaedic orthosis comprises: buckles (1); pins (2); adjuster guides (3); height-changing breathable calf support (4), preferably made of polyamide; angle adjuster (5); neutral angle guide (6); energy-accumulating flat upper spring section (7); screws (8), frame (9), preferably made of carbon fibre composite; energy-accumulating crescent-shaped bow (10); sole (or footplate) (11); calf straps (12); height adjustment holes (15) and frame holes (16). Number (13) shows the top view of a spring curve section cut and (14) shows the top view of the cut of spring and sole connection.

In the lower part of the frame on the outer side of the sole (11), the frame structure is uniformly formed into an energy-accumulating crescent-shaped bow (10), which, when directed ventrally, transitions uniformly into an energy-accumulating flat upper spring section (7). The height-changing breathable calf support (4) is attached to the frame with screws (8) through height adjustment holes (15) and frame holes (16) by using the angle adjuster (5) and neutral angle guide (6). The calf support (4) is attached to the leg with textile straps (12) which are passed through openings and buckles (1) in the calf support (4). When the orthosis is worn, the buckles (1) are fastened to the pins (2) integrated into the calf support (4).

The main parts of the orthosis, the height-changing breathable calf support (4), the carbon fibre composite frame (9), with the sole section (11), may be manufactured in different sizes and packaged as supports and frames in different proportions, assembled into a single orthosis system.

The sole (11) of the adjusted orthosis is adapted to the shape of the footwear worn by the individual according to his height and physical proportion, either by trimming with special scissors or by grinding it. After the sole has been adjusted and the orthosis has been inserted into the footwear worn, the person puts on the shoe. The orthosis is secured to the calf by means of textile straps (12), but these straps are left unattached at this stage of the adjustment. The suitability of the height-changing breathable calf support (4) and the carbon composite frame (9) for the height of the person is assessed. If the calf support of the orthosis is not obviously at an incorrect height, then the correct selection of the orthosis components has been made. The screws (8) of the calf support are loosened by unscrewing them slightly. The person stands up in his normal anatomical position. The angle of the height-changing breathable calf support (4) is adjusted by raising up or lowering down the angle adjuster (5) so that the calf support is evenly in contact with the ventral part of the person's calf. By raising or lowering the whole of the calf support (4), together with the angle adjuster (5), and the neutral angle guide (6), the precise adjustment of the height of the calf support is carried out.

If the angle of the person's calf is greater or less than the angle of the calf support at the maximum downward or upward angle of the angle adjuster (5), the angle adjuster (5) and the neutral angle guide (6) can be adapted by fully removing the screws (8) and sliding the angle adjuster (5) to its maximum upper position, and the neutral angle guide (6) - to the lowest lower position. These angle adjusting parts can be swapped by inserting the riser into the adjuster guides (3) at the bottom of the height-changing breathable calf support (4), and the neutral angle guide (6) into the top adjuster guides (3). After these steps, the adjustment of the angle and the height of the height-changing breathable calf support (4) is repeated by sliding the angle adjuster (5) and/or the entire calf support up or down.

Once the correct angle of the height-changing breathable calf support (4) has been adjusted, the screws (8) are tightened, thereby securing the height-changing breathable calf support (4) to the orthosis frame. The straps (12) are attached to the side of the buckles (1). The buckles (1) are fastened to the fixing pins (2) integrated into the height-changing breathable calf support (4) by passing the buckles through the pins and sliding them downwards. After the buckles have been fastened, the fastening straps (12) are tightened, cut and fixed in such a way that the height-changing breathable calf support (4) is firmly latched to the person's calf and prevents it from moving independently of the orthosis. The person is asked to walk for 10 minutes. If he does not feel any discomfort or significant pressure on the calf during the 10-minute period, the adjustment procedure is complete. Otherwise, the adjustment procedure is repeated.

When correctly fitted, adjusted and worn, the orthosis provides firm stabilisation of the ankle and knee joint in all phases of the stride, allowing the leg to move in only the biomechanically correct direction. During stepping, the energy generated between the sole (11) and the height-changing breathable calf support (4) in the carbon fibre composite frame is stored uniformly in energy accumulating flat upper spring section (7), located in a flat, coronal plane, and in the energy accumulating crescent-shaped bow (10) in the dorsal direction, while the excess energy is evenly distributed in the sole (11). At the end of the step phase, the energy stored in the energy accumulating flat spring section (7) and the energy accumulating crescent-shaped bow (10) is returned through the sole (11) and the height-changing breathable calf support (4), creating a cushioning effect and thus facilitating the completion of the step phase. Precise height and angle adjustment by means of angle adjuster (5) and neutral angle guide (6) and a height-changing breathable calf support (4), which also reduces perspiration, provide greater comfort. Specially designed buckles (1) with locking mechanisms facilitate the insertion and removal of the orthosis and ensure its reliable fastening.

## Claims

1. An ankle-foot orthopaedic orthosis comprising a carbon fibre composite frame (9), a height-changing breathable calf support (4) adapted to be attached to a leg, buckles (1) for attaching the breathable calf support (4) to the leg, where the height-changing breathable calf support (4) comprises a height adjustment mechanism allowing the height of the support to be adjusted in relation to the foot in the superior-inferior direction, and height adjustment holes (15) and fixing screws (8) which connect the height-changing breathable calf support (4) through frame holes (16) and said height adjustment holes (15) to said carbon fibre composite frame (9), where the frame (9) has an energy-accumulating flat spring section (7) in the coronal plane in the front part of the orthosis, **characterized in that** the frame (9) and the height-changing breathable calf support (4) are made of different materials to provide different wear characteristics, where the height-changing breathable calf support (4) is made of polyamide, and **in that** the height-changing breathable calf support (4) further comprises an angle adjuster (5) for allowing the angle to be adjusted in the dorsal-ventral direction, adjuster guides (3) and a neutral angle guide (6), said carbon fibre composite frame (9) further comprising a crescent-shaped bow (10), acting as a spring mechanism for energy storage, located on the lateral side of the foot in use, having an inner surface facing the foot of the user in use and an outer surface, said outer surface being concave and said inner surface being convex, where bending of the frame (9) takes place along said crescent-shaped bow (10) maintaining the uniformity of the shape and at the same time the uniformity of the stiffness of the frame (9) throughout its entire structure, wherein the crescent-shaped bow (10) is flattened at the sole connection (14) and rotates towards the front of the sole (11), and wherein the crescent-shaped bow (10) has a a ventral upward slope and a smooth transition to the energy-accumulating flat spring section (7) in the coronal plane in the front part of the orthosis.

2. The ankle-foot orthopaedic orthosis according to claim 1, **characterized in that** the height-changing breathable calf support (4) made of polyamide is flexible, has open-worked structure and shape of a butterfly's wings that adjusts to the calf over a wide range.

3. The ankle-foot orthopaedic orthosis according to claim 1, **characterized in that** the buckles (1) comprise two parts, one part having a fastening elements with a mushroom-shaped part (2) being directly incorporated into the structure of the height-changing breathable calf support (4) and forming part of the entirety of the orthosis calf support, and the other part, which attaches to the strap, having an opening for fastening with a flexible latch formed in one piece with this buckle part, which is centred in the direction of the calf strap and has a special crescent-shaped convex edge to facilitate unbuckling of the strap.

## Patentansprüche

1. Orthopädische Fußgelenkorthese, umfassend einen Kohlefaserverbundrahmen (9), eine höhenverstellbare, atmungsaktive Wadenstütze (4), die dazu geeignet ist, an einem Bein angebracht zu werden, Schnallen (1) zum Anbringen der atmungsaktiven Wadenstütze (4) am Bein, wobei die höhenverstellbare, atmungsaktive Wadenstütze (4) einen Höhenanpassungsmechanismus umfasst, er es ermöglicht, die Höhe der Stütze im Verhältnis zum Fuß in der superior-inferioren Richtung anzupassen, und Höhenanpassungslöcher (15) und Befestigungsschrauben (8), welche die höhenverstellbare, atmungsaktive Wadenstütze (4) durch Rahmenlöcher (16) und die Höhenanpassungslöcher (15) mit dem Kohlefaserverbundrahmen (9) verbinden, wobei der Rahmen (9) einen energiespeichernden flachen Federabschnitt (7) in der Koronalebene in dem vorderen Teil der Orthese aufweist,
**dadurch gekennzeichnet, dass** der Rahmen (9) und die höhenverstellbare, atmungsaktive Wadenstütze (4) aus unterschiedlichen Materialien bestehen, um unterschiedliche Verschleißkennzeichen bereitzustellen, wobei die höhenverstellbare, atmungsaktive Wadenstütze (4) aus Polyamid besteht, und dass die höhenverstellbare, atmungsaktive Wadenstütze (4) ferner eine Winkelanpassungsvorrichtung (5), damit der Winkel in der dorsoventralen Richtung angepasst werden kann, Anpassungsführungen (3) und eine neutrale Winkelführung (6) umfasst, wobei der Kohlefaserverbundrahmen (9) ferner eine sichelförmige Biegung (10) umfasst, die als Federmechanismus zur Energiespeicherung dient, sich im Gebrauch auf der lateralen Seite des Fußes befindet, eine innere Oberfläche, die dem Fuß des Benutzers im Gebrauch gegenübersteht, und eine äußere Oberfläche aufweist, wobei die äußere Oberfläche konkav ist und die innere Oberfläche konvex ist, wobei das Biegen des Rahmens (9) entlang der sichelförmigen Biegung (10) stattfindet, wobei die Einheitlichkeit der Form und gleichzeitig die Einheitlichkeit der Steifigkeit des Rahmens (9) über seine gesamte Struktur aufrechterhalten werden, wobei die sichelförmige Biegung (10) an der Sohlenverbindung (14) abgeflacht ist und sich in Richtung auf den Vorderteil der Sohle (11) dreht, und wobei die sichelförmige Biegung (10) eine ventrale nach oben gerichtete Neigung und einen glatten Übergang zu dem energiespeichernden flachen Federabschnitt (7) in der Koronalebene in dem vorderen Teil der Orthese aufweist.

2. Orthopädische Fußgelenkorthese nach Anspruch 1, **dadurch gekennzeichnet, dass** die höhenverstellbare, atmungsaktive Wadenstütze (4) aus Polyamid flexibel ist, eine offen angelegte Struktur und Form von Schmetterlingsflügeln aufweist, die sich über einen breiten Bereich an die Wade anpasst.

3. Orthopädische Fußgelenkorthese nach Anspruch 1, **dadurch gekennzeichnet, dass** die Schnallen (1) zwei Teile umfassen, wobei ein Teil Befestigungselemente mit einem pilzförmigen Teil (2) aufweist, der direkt in die Struktur der höhenverstellbaren, atmungsaktiven Wadenstütze (4) integriert ist und Teil der gesamten Orthesen-Wadenstütze ist, und der andere Teil, der an dem Riemen angebracht wird, eine Öffnung zum Befestigen mit einer flexiblen Zunge, die einstückig mit diesem Schnallenteil gebildet ist, aufweist, die in der Richtung des Wadenriemens zentriert ist und eine spezielle sichelförmige konvexe Kante aufweist, um das Abschnallen des Riemens zu erleichtern.

## Revendications

1. Orthèse orthopédique cheville-pied comprenant un cadre composite en fibre de carbone (9), un support de mollet respirant à hauteur variable (4) conçu pour être fixé à une jambe, des boucles (1) pour fixer le support de mollet respirant (4) à la jambe, où le support de mollet respirant à hauteur variable (4) comprend un mécanisme de réglage de la hauteur permettant de régler la hauteur du support par rapport au pied dans la direction supérieure-inférieure, et des trous de réglage de hauteur (15) et des vis de fixation (8) qui connectent le support de mollet respirant à hauteur variable (4) à travers des trous de cadre (16) et lesdits trous de réglage de hauteur (15) sur ledit cadre composite en fibres de carbone (9), où le cadre (9) a une section de ressort plat (7) accumulant de l'énergie dans le plan coronal dans la partie avant de l'orthèse,
**caractérisée en ce que** le cadre (9) et le support de mollet respirant à hauteur variable (4) sont constitués de différents matériaux pour présenter différentes caractéristiques d'usure, le support de mollet respirant à hauteur variable (4) est en polyamide, et **en ce que** le support de mollet respirant à hauteur variable (4) comprend en outre un dispositif de réglage d'angle (5) pour permettre de régler l'angle dans la direction dorso-ventrale, des guides de dispositif de réglage (3) et un guide d'angle neutre (6), ledit cadre composite en fibre de carbone (9) comprenant en outre un arc en forme de croissant (10), agissant comme un mécanisme à ressort pour le stockage d'énergie, situé sur le côté latéral du pied en cours d'utilisation, ayant une surface interne faisant face au pied de l'utilisateur en cours d'utilisation et une surface externe, ladite surface externe étant concave et ladite surface interne étant convexe, où s'effectue la flexion du cadre (9) le long dudit arc en forme de croissant (10) en maintenant l'uniformité de la forme et en même temps l'uniformité de la rigidité du cadre (9) sur toute sa structure, dans laquelle l'arc en forme de croissant (10) est aplati au niveau du raccord de semelle (14) et tourne vers l'avant de la semelle (11), et dans laquelle l'arc en forme de croissant (10) a une pente ventrale ascendante et une transition en douceur vers la section de ressort plat accumulant de l'énergie (7) dans le plan coronal dans la partie avant de l'orthèse.

2. Orthèse orthopédique cheville-pied selon la revendication 1, **caractérisée en ce que** le support de mollet respirant à hauteur variable (4) en polyamide est flexible, a une structure ajourée et une forme d'ailes de papillon qui s'adapte sur une large plage au mollet.

3. Orthèse orthopédique cheville-pied selon la revendication 1, **caractérisée en ce que** les boucles (1) comprennent deux parties, une partie ayant des éléments de fixation avec une partie en forme de champignon (2) étant directement incorporée dans la structure du support de mollet respirant à hauteur variable (4) et formant une partie de l'intégralité du support de mollet d'orthèse, et l'autre partie, qui s'attache à la sangle, ayant une ouverture de fixation avec un loquet souple formé d'une seule pièce avec cette partie de boucle, qui est centré dans la direction de la sangle de mollet et a un bord convexe spécial en forme de croissant pour faciliter le débouclage de la sangle.
